Europäisches Patentamt

European Patent Office

Office européen des brevets

(11) Veröffentlichungsnummer: **0 299 577 B1**

(19)

# EUROPÄISCHE PATENTSCHRIFT

(12)

(45) Veröffentlichungstag der Patentschrift: **08.01.92**

(51) Int. Cl.⁵: **C07C 29/70**, C07C 29/76, C07C 31/30

(21) Anmeldenummer: **88201457.4**

(22) Anmeldetag: **09.07.88**

(54) **Verfahren zur Herstellung von Alkoholaten.**

(30) Priorität: **14.07.87 DE 3723193**

(43) Veröffentlichungstag der Anmeldung: **18.01.89 Patentblatt 89/03**

(45) Bekanntmachung des Hinweises auf die Patenterteilung: **08.01.92 Patentblatt 92/02**

(84) Benannte Vertragsstaaten: **AT BE CH DE ES FR GB IT LI NL SE**

(56) Entgegenhaltungen: **WO-A-86/00819 DE-A- 3 526 755**

(73) Patentinhaber: **METALLGESELLSCHAFT AG Reuterweg 14 Postfach 3724 W-6000 Frankfurt/M.1(DE)**

(72) Erfinder: **Sander, Ulrich, Dr. Taunusstrasse 116 W-6382 Friedrichsdorf(DE)** Erfinder: **Soukup, Pavel Am Galgenberg 17 W-6451 Hammersbach(DE)** Erfinder: **Helmrich, Harald, Dr. Ben-Gurion-Ring 156 W-6000 Frankfurt am Main(DE)** Erfinder: **Weiss, Wilfried, Dr. Mühlenweg 41 W-6370 Oberursel 4(DE)**

## Beschreibung

Die Erfindung bezieht sich auf ein Verfahren zur Herstellung von Alkoholaten durch Umsetzung von Alkalihydroxiden und aliphatischen Alkoholen mit 1 bis 6 Kohlenstoffatomen bei einer Temperatur von 50 bis 110° C, vorzugsweise 80 bis 100° C, sowie einem Druck von 0,3 bis 1,2 bar. Als Rohstoffe für das Verfahren werden also LiOH, NaOH, KOH, RbOH und CsOH sowie Methanol, Äthanol, Propanol, Butanol, Pentanol und Hexanol verwendet, was natürlich die Verwendung aller isomeren Alkohole einschließt.

Bei der Bildung von Alkalialkoholaten nach der Gleichung ROH + MeOH $\rightleftharpoons$ ROMe + H2O, Me = Li, Na, K, Rb, Cs bildet sich Wasser, das aus dem Gleichgewicht entfernt werden muß, wenn ein weitgehend quantitativer Umsatz und/oder ein weitgehend wasserfreies Endprodukt angestrebt wird. Die Entfernung des gebildeten Wassers kann durch Destillation erfolgen, wobei allerdings dann Schwierigkeiten auftreten, wenn das Wasser mit dem Alkohol ROH ein Azeotrop bildet oder ein dem Alkohol ähnliches Verdampfungsverhalten hat. In beiden Fällen wird nämlich ein wasserhaltiges Stoffgemisch verdampft, das unbedingt in seine Bestandteile zu zerlegen ist. Dies stößt in vielen Fällen auf technische Schwierigkeiten bzw. verursacht erhebliche Kosten, so daß ein anderer Weg zur weitgehend quantitativen Abtrennung des Wassers beschritten werden muß.

Der Erfindung liegt daher die Aufgabe zugrunde, ein Verfahren zu schaffen, das es ermöglicht, das bei der Alkoholatbildung entstehende und immer neu gebildete Reaktionswasser ohne großen apparativen Aufwand schnell sowie nahezu quantitativ abzutrennen.

Die der Erfindung zugrundeliegende Aufgabe wird dadurch gelöst, daß man das bei der Reaktion gebildete dampfförmige Alkohol-Wasser-Gemisch über einen Tropfenabscheider führt, anschließend auf eine Temperatur aufheizt, die bis zu 50° C oberhalb der Reaktionstemperatur liegt, aber nicht größer als 120° C ist, danach durch mindestens eine Membran, die aus einem 50 bis 300 μm dicken Träger besteht, auf den eine mit Poren versehene, 50 bis 500 μm dicke Stützmembran aufgebracht ist, die wiederum eine 0,2 bis 5 μm dicke, porenfreie Trennschicht aufweist, in seine Bestandteile zerlegt, wobei zur Abtrennung von 1 kg Wasserdampf pro Stunde 20 bis 40 m², vorzugsweise 25 bis 35 m², Membranaustauscherfläche verwendet werden, und daß man schließlich den dampfförmigen Alkohol sowie das dampfförmige Wasser kondensiert und den Alkohol in die Umsetzung zurückführt. Die Erfindung ermöglicht es, daß kleine Wassermengen aus der Dampfphase mit hoher Selektivität und damit wirtschaftlich abgetrennt werden können, und der Vorteil des erfindungsgemäßen Verfahrens besteht darin, daß kurze Reaktionszeiten und hohe Ausbeuten erzielt werden.

Dadurch, daß man das bei der Alkoholatreaktion gebildete dampfförmige Alkohol-Wasser-Gemisch über einen Tropfenabscheider führt und anschließend auf eine Temperatur aufheizt, die bis zu 50° C oberhalb der Reaktionstemperatur liegt, aber nicht größer als 120° C ist, wird erreicht, daß mitgerissene alkali- bzw. alkoholathaltige Tröpfchen aus der Dampfphase sicher entfernt werden, wodurch die Lebensdauer der Membran verlängert wird, daß keine thermische Schädigung der Membran erfolgt und daß die Membran ausschließlich mit einer Dampfphase in Berührung ist, was sich im Langzeitbetrieb positiv auf die Trennleistung der Membran auswirkt. Die zur Durchführung des erfindungsgemäßen Verfahrens verwendete Membran hat eine sehr gute mechanische Festigkeit, was zu einer Erhöhung der Betriebssicherheit des erfindungsgemäßen Verfahrens beiträgt. Die nach der Erfindung zur Stofftrennung erforderliche spezifische Membranaustauschfläche gewährleistet, daß ein verhältnismäßig großer Dampfstrom mit hinreichender Geschwindigkeit und Selektivität in seine Bestandteile getrennt werden kann. Erst durch das Zusammenwirken der erfindungsgemäßen Merkmale kann die Membrantrenntechnik in ein Verfahren zur Herstellung eines Alkali-Alkoholats integriert werden, das im technischen Maßstab durchgeführt wird.

Nach der Erfindung ist es besonders vorteilhaft, wenn das Alkalihydroxid und der Alkohol im Molverhältnis 1 : 2 bis 1 : 10 eingesetzt werden, da durch dieses Verhältnis gewährleistet ist, daß während der gesamten Reaktionszeit eine genügend große Alkoholmenge vorhanden ist, die bei ihrer Verdampfung das ständig nachgebildete Wasser abführt. Die zur Durchführung des erfindungsgemäßen Verfahrens verwendete Membran hat bezüglich ihrer Alterungsbeständigkeit und Selektivität besonders vorteilhafte Eigenschaften, wenn der Träger aus einem Polyester-Gewebe oder -Faservlies, die Stützmembran aus Polyacrylnitril oder einem Polysulfon und die Trennschicht aus Polyvinylalkohol besteht, wobei der Durchmesser der Poren der Stützmembran vom Träger zur Trennschicht abnimmt. Die an die Trennschicht angrenzenden Poren der Stützmembran haben einen Durchmesser von 0,05 bis 10 μm.

Aus den Druckschriften US-PS 4 405 409 und Chem.-Ing.-Tech. 58, 1986, Seiten 740 bis 742, ist zwar an sich bekannt, daß Stoffgemische beim Durchgang durch Membranen in ihre Bestandteile getrennt werden können; der Fachmann konnte aus diesen Druckschriften aber nicht herleiten, daß dampfförmige Stoffgemische in ihre Bestandteile zerlegt werden können, denn in beiden Veröffentlichungen wird vorgeschlagen, daß der Membran ein flüssiges Stoffgemisch zugeführt werden soll, das in eine durch die Membran hindurchtretende dampfförmige Komponente und eine von der Membran abfließende flüssige Komponente getrennt wird. Auch die Tatsache, daß aus der DE-OS 35 26 755 ein Verfahren zur Trennung azeotroper Zweistoff-Gemische bekannt ist, bei dem in einer ersten mehrstufigen Rektifikationseinrichtung aus dem Zweistoff-Gemisch · ein vorkonzentriertes, dampfförmiges Zwischenprodukt gewonnen wird, bei dem dieses dampfförmige Zwischenprodukt anschließend überhitzt und in einer mehrstufigen Pervaporationseinrichtung zu einem dampfförmigen Vorprodukt aufkonzentriert wird und bei dem schließlich aus diesem Vorprodukt in einer zweiten mehrstufigen Rektifikationseinrichtung die abzutrennende Komponente bis auf den im Endprodukt zulässigen Gehalt ausgetrieben wird, konnte den Fachmann nicht zur Schaffung des erfindungsgemäßen Verfahrens veranlassen, denn er mußte davon ausgehen, daß die in der Dampfphase befindlichen geringen Alkali-Alkoholatmengen, die zur Trennung der Dampfphase verwendeten Membranen zumindest während des Langzeitbetriebs so schädigen, daß die Pervaporation sich im technischen Maßstab nicht in ein Verfahren zur Herstellung von Alkali-Alkoholaten integrieren läßt. Es ist das Verdienst der vorliegenden Erfindung, durch die Kombination mehrerer Merkmale die nachteilige Wirkung des bei der Alkali-Alkoholatherstellung immer in geringer Menge in der Dampfphase vorhandenen Alkali-Alkoholats auf die Lebensdauer der Membran vermieden zu haben, so daß die Membrantechnik auch bei der Herstellung von Alkali-Alkoholaten zur Anwendung kommen kann. Überraschenderweise hat sich nämlich gezeigt, daß die bei der Alkoholatbildung anfallende Dampfphase durch Pervaporation in ihre Bestandteile zerlegt werden kann, ohne daß die zur Pervaporation benötigten Membranen durch Alkalihydrocide, die sich durch Hydrolyse der in die Dampfphase übergegangenen Alkali-Alkoholate bilden, geschädigt werden.

Die Reinheit der nach dem erfindungsgemäßen Verfahren hergestellten Alkoholate ist dann sehr hoch, wenn die Alkalihydroxide frei von Sauerstoff und Peroxiden sind, wenn die Alkohole sauerstofffrei sowie frei von Aldehyden und Ketonen sind und wenn die in den Prozeßapparaten enthaltene Atmosphäre frei von Sauerstoff ist. Der Alkalihydroxidgehalt der Alkoholate ist dadurch weiter zu vermindern, daß die Alkoholate durch Umkristallisieren gereinigt werden, wobei als Lösungsmittel der Alkohol verwendet wird, der auch im Alkalialkoholat enthalten ist. Die Kondensation der dampfförmigen Alkohole und des Wasserdampfs kann entweder unmittelbar hinter der Membran oder aber in einem besonderen Kondensator erfolgen, wobei durch die Kondensation der dampfförmigen Alkohole eine Druckminderung eintritt. Der Unterdruck kann zur Einstellung des bei der Alkoholatbildung einzuhaltenden Reaktionsdrucks eingesetzt werden. Das erfindungsgemäße Verfahren kann sowohl diskontinuierlich als auch kontinuierlich betrieben werden. Beim diskontinuierlichen Betrieb erfolgt die Verdampfung des Alkohols bis zu dem Punkt, an dem die gewünschte Alkoholatkonzentration im Reaktionsgemisch vorliegt. Beim kontinuierlichen Betrieb wird das Reaktionsgemisch durch mehrere Reaktoren geführt, in denen unterschiedliche Reaktionsbedingungen herrschen, so daß die Alkoholatkonzentration von Reaktor zu Reaktor ansteigt. Der letzte Reaktor kann als Sprühtrockner gestaltet werden, in dem die restlichen Alkoholmengen aus dem Alkoholat entfernt werden. Das erfindungsgemäße Verfahren liefert Alkoholate in sehr hoher Ausbeute und von großer Reinheit.

Das erfindungsgemäße Verfahren wird nachfolgend anhand eines Ausführungsbeispiels näher erläutert.

Eine Reaktor wurde mit 18 kg KOH und 82 kg $C_2H_5OH$ beschickt. Das KOH hatte einen Wassergehalt von ca. 10 Gew.%, und es war frei von Kaliumperoxid sowie Sauerstoff. Das verwendete Äthanol wurde vor seinem Einsatz durch Spülen mit Stickstoff vom Sauerstoff befreit und enthielt keine Aldehyde. Vor der Beschickung des Reaktors mit den Rohstoffen wurde der Sauerstoff aus dem Reaktionsraum durch Spülen mit Stickstoff entfernt.

Die Alkoholatbildung erfolgte während einer Zeit von 4 Stunden bei einer Reaktionstemperatur von ca. 80° und einem Reaktionsdruck von ca. 0.9 bar. Während der Alkoholatbildung verdampften Äthanol und Wasser, wobei die verdampfte Wassermenge innerhalb der Reaktionszeit abnahm. Das dampfförmige Äthanol-Wasser-Gemisch wurde aus dem Reaktor in einen Tropfenabscheider geführt, wo die bei der Verdampfung mitgerissenen Tröpfchen des Reaktionsgemisches zurückgehalten wurden. Anschließend gelangte die Dampfphase in einen Überhitzer, wo eine Temperatur von 100° C eingestellt wurde. Danach wurde die überhitzte Dampfphase einem Pervaporator zugeführt, der eine Membrantrennfläche von 240 m² aufwies. Der in der Dampfphase enthaltene Wasserdampf passierte die Membran, während das dampfförmige Äthanol von der Membran zurückgehalten wurde und aus dem Pervaporator austrat. Das wasserfreie dampfförmige Äthanol gelangt in einen Kondensator, wo sich durch Verflüssigung des Äthanols ein Unterdruck einstellte, der zur Regelung des Reaktionsdrucks herangezogen wurde. Das flüssige Äthanol wurde einem Vorratsbehälter zugeführt und erneut zur Alkoholatherstellung verwendet. Das durch die Membran abgetrennte Wasser wurde kondensiert und verworfen. Nach Beendigung der Reaktion lag im Reaktor ein flüssiges Produkt vor, das aus 19,7 Gew.% $C_2H_5OK$, 0,7 Gew.% KOH und Rest $C_2H_5OH$ besteht. Das flüssige Produkt war farblos und enthielt keine organischen Verunreinigungen.

## Patentansprüche

1. Verfahren zur Herstellung von Alkoholaten durch Umsetzung von Alkalihydroxiden und aliphatischen Alkoholen mit 1 bis 6 Kohlenstoffatomen bei einer Temperatur von 50 bis 110°C, vorzugsweise 80 bis 100°C, sowie einem Druck von 0,3 bis 1,2 bar, dadurch gekennzeichnet, daß man das bei der Reaktion gebildete dampfförmige Alkohol-Wasser-Gemisch über einen Tropfenabscheider führt, anschließend auf eine Temperatur aufheizt, die bis zu 50°C oberhalb der Reaktionstemperatur liegt, aber nicht größer als 120°C ist, danach durch mindestens eine Membran, die aus einem 50 bis 300μm dicken Träger besteht, auf den eine mit Poren versehene, 50 bis 500 μm dicke Stützmembran aufgebracht ist, die wiederum eine 0,2 bis 5 μm dicke, porenfreie Trennschicht aufweist, in seine Bestandteile zerlegt, wobei zur Abtrennung von 1 kg Wasserdampf pro Stunde 20 bis 40 m², vorzugsweise 25 bis 35 m², Membranaustauscherfläche verwendet werden, und daß man schließlich den dampfförmigen Alkohol sowie das dampfförmige Wasser kondensiert und den Alkohol in die Umsetzung zurückführt.

2. Verfahren nach Anspruch 1, dadurch gekennzeichnet, daß man das Alkalihydroxid und den Alkohol im Molverhältnis 1 : 2 bis 1 : 10 einsetzt.

3. Verfahren nach den Ansprüchen 1 bis 2, dadurch gekennzeichnet, daß der Träger aus einem Polyester-Gewebe oder -Faservlies, die Stützmembran aus Polyacrylnitril oder einem Polysulfon und die Trennschicht aus Polyvinylalkohol besteht, wobei der Durchmesser der Poren der Stützmembran vom Träger zur Trennschicht abnimmt.

## Claims

1. Process for producing alcoholates by reacting alkali metal hydroxides and aliphatic alcohols with 1 to 6 carbon atoms at a temperature of 50 to 110°C, preferably 80 to 100°C, and at a pressure of 0.3 to 1.2 bar, characterised in that the vaporous alcohol/water mixture formed during the reaction is passed over a mist eliminator, then heated to a temperature which is up to 50°C above the reaction temperature, but is not greater than 120°C, then is decomposed into its constituents through at least one membrane, which consists of a carrier 50 to 300 μm thick, to which carrier is applied a supporting membrane 50 to 500 μm thick which is provided with pores and which itself has a nonporous separating layer 0.2 to 5 μm thick, whereby 20 to 40 m², preferably 25 to 35 m², membrane exchange area are used to separate off 1 kg water vapour per hour, and that finally the vaporous alcohol and the vaporous water are condensed and the alcohol is recycled into the reaction.

2. Process according to Claim 1, characterised in that the alkali metal hydroxide and the alcohol are used in a molar ratio of 1 : 2 to 1 : 10.

3. Process according to Claims 1 to 2, characterized in that the carrier consists of a woven polyester fabric or non-woven polyester fibre fabric, the supporting membrane consists of polyacrylonitrile or a polysulphone and the separating layer consists of polyvinyl alcohol, the diameter of the pores of the supporting membrane decreasing from the carrier to the separating layer.

## Revendications

1. Procédé de préparation d'alcoolates par réaction d'hydroxydes de métal alcalin et d'alcools aliphatiques ayant de 1 à 6 atomes de carbone, à une température de 50 à 110°C et, de préférence, de 80 à 100°C, ainsi que sous une pression de 0,3 à 1,2 bar, caractérisé en ce qu'il consiste à envoyer le mélange d'alcool et d'eau, sous forme de vapeur, formé lors de la réaction, sur un séparateur de gouttes, à le porter ensuite à une température qui est supérieure de jusqu'à 50°C à la température de réaction, mais qui n'est pas supérieure à 120°C, à le fractionner ensuite en ses constituants par au moins une membrane qui est constituée d'un support de 50 à 300 μm d'épaisseur, sur lequel est déposée une membrane d'appui munie de pores, et de 50 à 500 μm d'épaisseur, laquelle comporte à son tour une couche de séparation de 0,2 à 5 μm d'épaisseur et exempte de pores en utilisant, pour séparer 1 kg de vapeur d'eau/heure, de 20 à 40 m² et, de préférence, de 25 à 35 m² de surface échangeuse de membrane, et à condenser enfin l'alcool sous forme de vapeur ainsi que l'eau sous forme de vapeur et à retourner l'alcool à la réaction.

2. Procédé suivant la revendication 1, caractérisé en ce qu'il consiste à utiliser l'hydroxyde de métal alcalin et l'alcool en le rapport molaire de 1:2 à 1:10.

3. Procédé suivant la revendication 1 ou 2, caractérisé en ce que le support est un tissu ou un non tissé fibreux de polyester, la membrane d'appui étant polyacrylonitrile ou en une polysulfone et la couche de séparation est en alcool polyvinylique, le diamètre des pores de la membrane d'appui diminuant du support vers la couche de séparation.